# EUROPEAN PATENT SPECIFICATION

(11) **EP 2 637 651 B1**
(45) Date of publication and mention of the grant of the patent: **20.01.2016**
(21) Application number: 11810884.4
(22) Date of filing: 10.11.2011
(51) Int. Cl.: A61K 31/07, A61K 31/122, A61K 45/06, A61P 27/00, A61P 1/00

(54) **COMPOSITION FOR TREATING PHONATORY AND OLFACTORY APPARATUS DISORDERS**
ZUSAMMENSETZUNG ZUR BEHANDLUNG VON ERKRANKUNGEN DER HÖHR- UND RIECHORGANE
COMPOSITION POUR LE TRAITEMENT DE TROUBLES DE L'APPAREIL PHONATOIRE ET OLFACTIF

(30) Priority: 12.11.2010 IT MI20102100
(43) Date of publication of application: 18.09.2013
(73) Proprietor: Scharper S.p.A., 20139 Milano (IT)
(72) Inventor: CARTOLARI, Marco, I-20900 Monza (IT)
(74) Representative: Villa, Livia
(86) International application number: PCT/IB2011/055015
(87) International publication number: WO 2012/063217

(56) References cited:
- EP-A1- 1 842 557
- CN-A- 1 310 998
- JP-A- 2007 204 436

## Description

### FIELD OF THE INVENTION

The present invention relates to a composition consisting of coenzyme Q10, vitamin A and suitable excipients for treating disorders of the phonatory and olfactory apparatus.

### PRIOR ART

Abuse or misuse of the voice can cause pathological processes, for example temporary or chronic inflammatory processes, which may mainly affect the mucosa and the musculature of the larynx and, in particular, that of the vocal cords. They are situations caused by vocal stress that is particularly violent or protracted.

The early symptoms indicative of vocal problems to which attention is to be paid are dysphonia, phonasthenia (change in one's voice over the course of the day), sensation of lack of air, frequent need to clear one's throat, feeling of tension and/or tenderness of the neck, cough and hoarseness, episodes of loss of voice (aphonia) with some difficulty of recovery, sensation of inadequacy of one's own vocal volume, difficulty in reaching vocal extensions that were comfortable before (in the context of singing, including amateur), difficulty swallowing (dysphagia).

In particular situations, characterized by abuse of the voice, the vocal cords can form polyps or nodules, usually always in the same position, exactly as occurs for the formation of calluses in areas of skin more exposed to external traumatic factors.

Specifically, there are conditions in which abuse or misuse of the voice can cause, through a mechanism of hyperaemia, a chronic inflammatory process, which leads to the formation of laryngeal nodules. It should also be considered likely that changes in muscular dynamics such as in the case of phonatory fatigue, following said abuse of the voice, results in a muscular change that also causes dysphonia. A vocal nodule represents the clinical result of altered muscular dynamics and of changes in elasticity and of the sliding of the vocal cords. It is often the point of contact of a phonatory posture in which the vocal cords are disposed on a plane with upper concavity, caused by muscular hypotonia.

A previous vocal nodule can evolve into a vocal polyp such as to affect the mucosal corium with variable representation of histological components (vascular and connective).

Medical treatment is known for these phonatory disorders only and exclusively of the logopaedic type, which envisages cycles of phoniatric therapy by avoiding phonatory abuse, making possible a gradual reduction of the nodular formation.

In the area of olfactory disorders, with transient or permanent dysosmia, there is a change in the ability to perceive one or more odours. Dysosmia can involve complete loss of olfaction (anosmia) or partial loss (hyposmia).

The European patent application EP 1 842 557 discloses the use of vitamin A for treating disorders of the vocal cord and larynx such as vocal cord scarring, vocal cord mucosal fibrosis, and laryngeal fibrosis.

The Japanese patent application JP 2007 204436A discloses the oral use of coenzyme Q10 for improving uttering a high-pitched sound region or a low-pitched sound region reasonably and without hurting vocal cord in phonation.

The object of the present invention is to find a product that is effective for the aforementioned applications, but which at the same time makes it possible to improve the overall result following application of the known treatments.

### SUMMARY OF THE INVENTION

The abovementioned object was achieved by a composition consisting of coenzyme Q10, Vitamin A and pharmaceutically acceptable excipients.

In another aspect, the present invention relates to the use of this composition for treating phonatory and olfactory apparatus disorders, for example anosmia and dysosmia.

For the purposes of the present invention, by the term "vitamin A" it is meant both the free form vitamin A, i.e. retinol, and derivatives thereof, such as retinaldehyde, retinoic acid, retinyl retinoate, a salt thereof, such as sodium retinoate, an ester thereof, such as retinyl acetate, retinyl propionate, retinyl palmitate, retinyl stearate, retinyl oleate, methyl retinoate, ethyl retinoate, or a mixture thereof.

### BRIEF DESCRIPTION OF THE DRAWINGS

The characteristics and advantages of the present invention will be clear from the detailed description given below, from the illustrative, non-limiting examples, and from the appended drawings, wherein:
- Fig. 1 shows the percentage *Jitter* pre-treatment and post-treatment with the composition of the present invention as per Example 9; and
- Fig. 2 shows the percentage *Shimmer* before and after treatment with the composition of the present invention as from Example 9.

### DETAILED DESCRIPTION OF THE INVENTION

The invention therefore relates to a composition consisting of coenzyme Q10, vitamin A and pharmaceutically acceptable excipients.

In fact, it has been surprisingly found that this composition is particularly effective in treating disorders of both the phonatory and olfactory apparatus, such as anosmia and dysosmia, as will be demonstrated in the examples presented below. Coenzyme Q10, also called ubiquinone (or vitamin Q) is an organic molecule, and more precisely a benzoquinone having a side chain with 10 isoprene units. Structurally, it is similar to vitamin K and vitamin E.

Preferably, said vitamin A is retinol, retinaldehyde, retinoic acid, retinyl retinoate, sodium retinoate, retinyl acetate, retinyl propionate, retinyl palmitate, retinyl stearate, retinyl oleate, methyl retinoate, ethyl retinoate, or a mixture thereof. Preferably, in the composition of the invention, coenzyme Q10 is in an amount up to 30 wt.%, and vitamin A is in an amount up to 12 wt.%, on the composition weight.

More preferably, in the composition of the invention, coenzyme Q10 is in an amount of 1-25 wt.%, and vitamin A 0.3-10 wt.%, based on the weight of said composition.

Even more preferably, in the composition of the invention, coenzyme Q10 is in an amount of 2-20 wt.%, and vitamin A 0.5-8 wt.%, on the composition weight. According to a particularly preferred embodiment, in the composition of the invention, coenzyme Q10 is in an amount of 3-15 wt.%, and vitamin A 1-6 wt.%, on the composition weight.

A composition wherein coenzyme Q10 is in an amount of 5-10 wt.%, and vitamin A 1.5-5 wt.%, on the composition weight, is further preferred.

Alternatively, a composition is preferred wherein coenzyme Q10 is in an amount of 5-10 wt.%, and vitamin A 0.3-2.5 wt.%, based on the weight of the composition. According to a preferred embodiment of the composition of the invention, the amount of coenzyme Q10 is higher than the amount of vitamin A.

As far as the pharmaceutically acceptable excipients are concerned, these are diluents, disintegrants, glidants, binders, lubricants, stabilizers, adsorbents, preservatives, controlled release agents and mixtures thereof. Preferably, said excipients are chosen from colloidal silica, dibasic calcium phosphate, maltodextrins, sugar esters, microcrystalline cellulose, lactose, talc, magnesium stearate, calcium stearate, aluminium silicate or magnesium silicate, and mixtures thereof. More preferably, said excipients are chosen from colloidal silica, dibasic calcium phosphate, maltodextrins, sugar esters, and mixtures thereof. The term "sugar esters" means sucrose monopalmitate, sucrose monostearate, sucrose dipalmitate, sucrose distearate, sucrose alkylate and mixtures thereof.

According to a preferred embodiment, the composition of the invention consists of:
- 6wt.% of coenzyme Q10,
- 2.1 wt.% of vitamin A, and
- 91.9 wt.% of a mixture of colloidal silica, dibasic calcium phosphate, maltodextrins, and sugar esters.

According to another preferred embodiment, the composition of the invention consists of:
- 9 wt.% of coenzyme Q10,
- 2.1 wt.% of vitamin A, and
- 89.9 wt.% of a mixture of colloidal silica, dibasic calcium phosphate, maltodextrins, and sugar esters.

According to a further preferred embodiment, the composition of the invention consists of:
- 9 wt.% of coenzyme Q10,
- 0.3 wt.% of vitamin A, and
- 90.7 wt.% of a mixture of colloidal silica, dibasic calcium phosphate, maltodextrins, and sugar esters.

The composition of the invention can be in the form of capsule, tablet, mini-tablet, micro-tablet, granule, micro-granule, pellet, multiparticulate, or micronized particulate. Preferably, it is in the form of capsule.

In another aspect, the present invention relates to the therapeutic application of the composition described above.

In particular, the composition of the invention is used in the treatment of phonatory and olfactory apparatus disorders, for example anosmia and dysosmia. "Phonatory apparatus disorders" means dysphonia, cordal hypotonia, phonatory fatigue, inflammatory states, nodules and polyps. As will in fact be seen from the examples given below, the composition of the invention has displayed a surprising synergistic effect ascribable to the suitable combination of the various components, which when used separately did not show suitable efficacy for the purposes of the present invention.

It has been, in fact, observed that coenzyme Q10 exerts a substantial action in the cells of the body, in that it permits production of energy and in particular synthesis of ATP (adenosine triphosphate) mainly in the muscles; a deficiency of coenzyme Q10 has the effect that the muscle fibres become more easily exhausted with clinical manifestations of hypotonia and muscular weakness. Moreover, coenzyme Q10 also exerts antioxidant and protective action against the production of free radicals, which represent one of the major causes of damage to cellular structures. For its part, vitamin A is an essential element in the formation and protection of the epithelial tissues in general (tissues with the function of internal and external coating of the surfaces of the body) and in maintaining conditions of well-being of the mucous membranes (organs coating surfaces communicating with the exterior). It is, moreover, necessary for protein synthesis at cellular level.

The composition of the present invention, moreover, can be used advantageously as adjuvant in treatments for logopaedic rehabilitation.

In this case, in fact, a suitable combination of coenzyme Q10 and vitamin A has been found to contribute to the prevention and reduction of muscular damage and restoration of the concentration of intracellular energy substances (ATP) necessary for proper functional capacity of the muscles of the vocal cords. Through its action on the epithelium of the vocal cords (in particular on the stellate cells), the composition was moreover found to contribute to the restoration and maintenance of the viscoelastic properties (elasticity and lubrication) of the whole vocal cord. The neuroprotective activity exerted by coenzyme Q10 contributes, together with vitamin A, to promoting functional recovery of the neurosensory network assigned to olfactory functionality.

Preferably, said composition is to be administered by the oral route. Alternatively, it can be administered parenterally after dilution in a suitable diluent. Moreover, other routes of administration are also possible, such as inhalation, aerosol, spray, transdermic, intravenous, intramuscular, transdermal, subcutaneous, intraperitoneal, intranasal, intrarectal, sublingual or topical.

Preferably, the composition of the invention is in the form of a unit dose comprising 3.3-83 mg of coenzyme Q10 and 1-26.6 mg of vitamin A.

More preferably, the composition of the invention is in the form of a unit dose comprising 15-35 mg of coenzyme Q10 and 5-15 mg of vitamin A.

Alternatively, the composition is preferred in the form of a unit dose comprising 15-35 mg of Coenzyme Q10 and 1-6.7 mg of vitamin A.

According to a preferred embodiment, said unit dose consists of:
- 20 mg of coenzyme Q10,
- 7 mg of vitamin A, and
- 305 mg of a mixture of colloidal silica, dibasic calcium phosphate, maltodextrins, and sugar esters.

According to another preferred embodiment, said unit dose consists of:
- 30 mg of coenzyme Q10,
- 7 mg of vitamin A, and
- 295 mg of a mixture of colloidal silica, dibasic calcium phosphate, maltodextrins, and sugar esters.

According to a further preferred embodiment, said unit dose consists of:
- 30 mg of coenzyme Q10,
- 1 mg of vitamin A, and
- 301 mg of a mixture of colloidal silica, dibasic calcium phosphate, maltodextrins, and sugar esters.

The composition and the unit dose of the present invention are prepared by mixing the compounds in the proportions by weight defined above.

According to a preferred embodiment, said unit doses are administered in an amount of 1-2 doses per day, more preferably for a period of time between 10 consecutive days and several consecutive months. In the case of chronic pathologies, said dose could be taken in cycles of 15-25 consecutive days every 1-2 months.

Illustrative, non-limiting examples of carrying out the present invention are presented below.

### EXAMPLES

### Example 1

According to the present invention, 332 g of a composition was prepared consisting of:

| | Quantity (mg) |
|---|---|
| Coenzime Q10 | 20 |
| Vitamin A⁽¹⁾ | 7 |
| Colloidal silica | 5 |
| Dibasic calcium phosphate | 120 |
| Maltodextrins | 160 |
| Sugar esters | 20 |

| | |
|---|---|
| ⁽¹⁾ retinyl acetate | |

### Example 2

According to the present invention, 332 g of a composition was prepared consisting of:

| | Quantity (mg) |
|---|---|
| Coenzime Q10 | 30 |
| Vitamin A⁽¹⁾ | 7 |
| Colloidal silica | 5 |
| Dibasic calcium phosphate | 20 |
| Maltodextrins | 240 |
| Sugar esters | 30 |

| | |
|---|---|
| ⁽¹⁾ retinyl acetate | |

### Example 3

According to the present invention, 332 g of a composition was prepared consisting of:

| | Quantity (mg) |
|---|---|
| Coenzime Q10 | 50 |
| Vitamin A⁽¹⁾ | 15.5 |
| Colloidal silica | 100 |
| Dibasic calcium phosphate | 166.5 |

| | |
|---|---|
| ⁽¹⁾ retinyl acetate | |

### Example 4

According to the present invention, 332 g of a composition was prepared consisting of:

| | Quantity (mg) |
|---|---|
| Coenzime Q10 | 10 |
| Vitamin A⁽²⁾ | 1.5 |
| Microcrystalline cellulose | 115 |
| Magnesium stearate | 125 |
| Talc | 80.5 |

| | |
|---|---|
| ⁽²⁾ retinol | |

### Example 5

In accordance with the present invention, 332 g of a composition was prepared consisting of:

| | Amount (mg) |
|---|---|
| Coenzyme Q10 | 30 |
| Vitamin A⁽²⁾ | 1 |
| Colloidal silica | 5 |
| Dibasic calcium phosphate | 23 |
| Maltodextrins | 240 |
| Sugar esters | 33 |

| | |
|---|---|
| ⁽²⁾ retinol | |

### Example 6

In accordance with the present invention, 332 g of a composition was prepared consisting of:

| | Amount (mg) |
|---|---|
| Coenzyme Q10 | 70 |
| Vitamin A⁽³⁾ | 12 |
| Colloidal silica | 10 |
| Dibasic calcium phosphate | 3 |
| Maltodextrins | 224 |
| Sugar esters | 13 |

| | |
|---|---|
| ⁽³⁾ sodium retinoate | |

### Example 7

In accordance with the present invention, 332 g of a composition was prepared consisting of:

| | Amount (mg) |
|---|---|
| Coenzyme Q10 | 40 |
| Vitamin A⁽⁴⁾ | 10 |
| Colloidal silica | 32 |
| Dibasic calcium phosphate | 30 |
| Maltodextrins | 200 |
| Sugar esters | 20 |

| | |
|---|---|
| ⁽⁴⁾ retinyl oleate | |

### Example 8

In accordance with the present invention, 332 g of a composition was prepared consisting of:

| | Amount (mg) |
|---|---|
| Coenzyme Q10 | 10 |
| Vitamin A⁽⁵⁾ | 5 |
| Colloidal silica | 50 |
| Dibasic calcium phosphate | 7 |
| Maltodextrins | 250 |
| Sugar esters | 10 |

| | |
|---|---|
| ⁽⁵⁾ ethyl retinoate | |

### Example 9

### Evaluation of efficacy of the composition of the invention

Gelatin capsules were prepared, each containing the composition obtained according to Example 2.

These capsules were then administered at a dose of two a day for a period of 12 consecutive days to 10 individuals, suitably selected.

The evaluation is presented below, divided into two phases:
- Phase I: pre-treatment with the composition of the invention; and
- Phase II: post-treatment with the composition of the invention.

### PHASE I

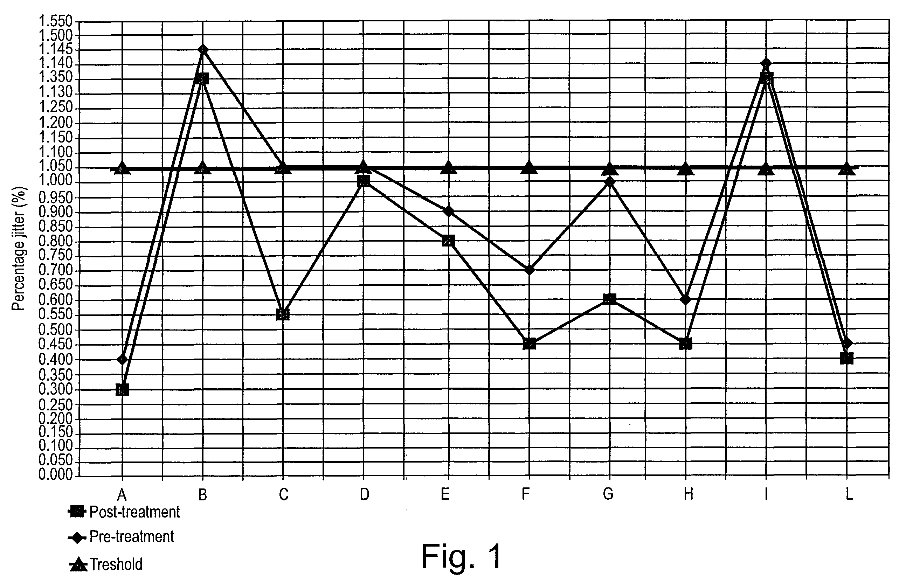

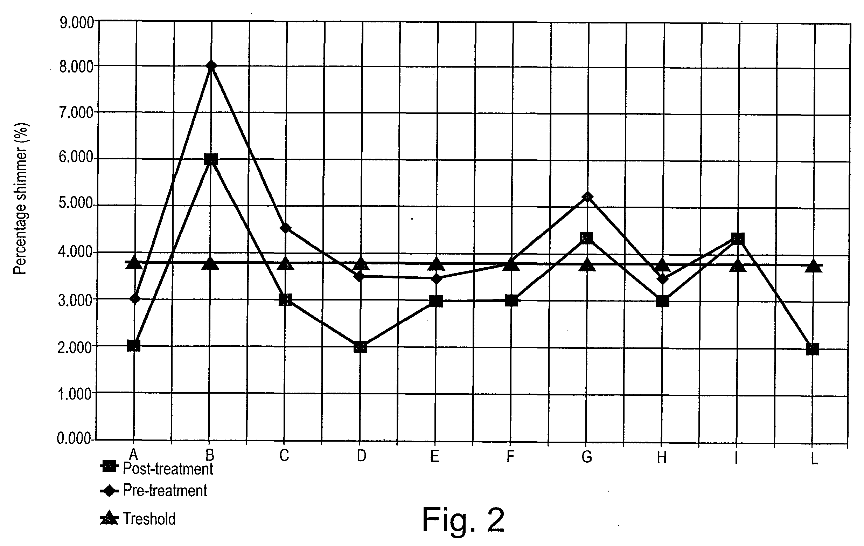

1) Logopaedic evaluation, comprising the phases of:
   a) taking a brief anamnesis of the 10 individuals, with particular attention to the presence of gastro-oesophageal reflux, allergic pathology and cigarette smoking;
   b) evaluation of tone of the respiratory muscles;
   c) evaluation of respiratory dynamics;
   d) evaluation of the duration of vocalization of "ah";
   e) perceptive evaluation of the quality of the voice; and
   f) compilation of the GIRBAS scale⁽*⁾.
   ⁽*⁾ GIRBAS scale (acronym for *Grade, Instability,* Roughness, *Breathiness, Asthenia, Strain):*
   0 = normal
   1 = slight deterioration
   2 = moderate deterioration
   3 = severe deterioration
   Referring to Table 1 below, analysis of the data obtained identified that 5 individuals presented inadequate parameters (individuals B, C, F, G, I), while the remaining 5 individuals were adequate (individuals A, D, E, H, L). 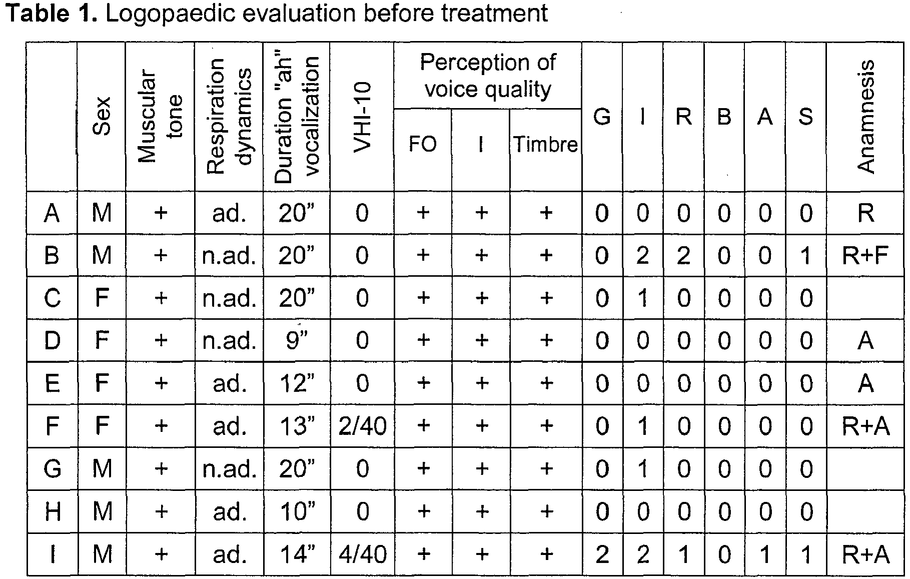 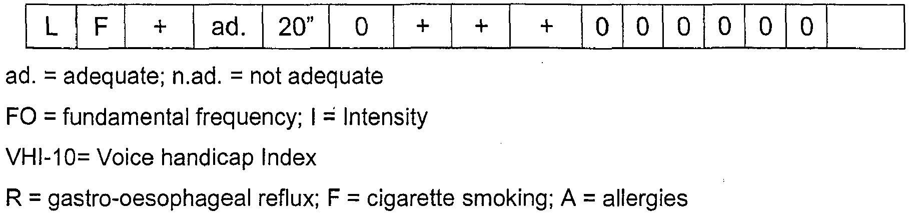
2) Evaluation of vocal quality using the Multi-Dimensional Voice Program (MDVP)
   Referring to the drawings, Fig. 1 represents the percentage *Jitter,* i.e. the average relative period-to-period short-term variability of the fundamental period. It is an indicator of irregularity of glottic vibration: the higher its percentage value, the hoarser the individual's voice.
   In its turn, Fig. 2 shows the percentage *Shimmer,* i.e. the average relative period-to-period short-term variability of the peak-to-peak amplitude. Also this parameter is an indicator of irregularity of glottic vibration: the higher its percentage value, the more the individual's voice is husky and hoarse.
   Finally, the *Soft Phonation Index* (SPI) was measured. This parameter is inversely proportional to the force of adduction of the vocal cords. In fact, the higher the force of adduction, the lower the value of SPI, because there is greater richness of high-frequency harmonics.
   From the results obtained, it was observed that:
   - in 5 individuals with inadequate vocal parameters, the Jitter values were altered in 2 individuals/5 ("B" and "I"), at the upper limits of the normal range in 2 individuals/5 ("C" and "G") 2 normal in 1 individual/5 ("F"); the Shimmer values were altered in 4 individuals/5 ("B", "C", "G", I"), at the upper limits of the normal range in 1 individual/5 ("F") and normal in 0 individuals. The values of SPI were found to be normal in 5 individuals/5 ("B", "C", "F", "G", "I").
   - in 5 individuals for whom adequate vocal parameters were observed, the Jitter values were altered in 0 individuals, at the upper limits of the normal range in 1 individual/5 ("D") and normal in 4 individuals/5 ("A", "E", "H", "I"); the Shimmer values were altered in 0 individuals, at the upper limits of the normal range in 3 individuals/5 ("D", "E", "H") and normal in 2 individuals/5 ("A", "I"). The values of SPI were at the upper limits of the normal range in 1 individual/5 ("H") and 4 individuals normal ("A", "D", "E", "L").

### PHASE II

### 1) Logopaedic evaluation

Referring to Table 2 below, analysis of the individuals shows that for the 5 individuals whose voice parameters proved to be inadequate, individual "I" did not have improvements, remaining stable, individual "B" had an improvement of the vocal parameters, but without normalization thereof, individuals "C", "F" and "G" saw normalization of their vocal parameters.

In conclusion, after administration of the composition of the invention, 8 individuals/10 had adequate vocal parameters, 1 individual/10 had improved but inadequate vocal parameters, and only one individual/10 did not show significant improvements.

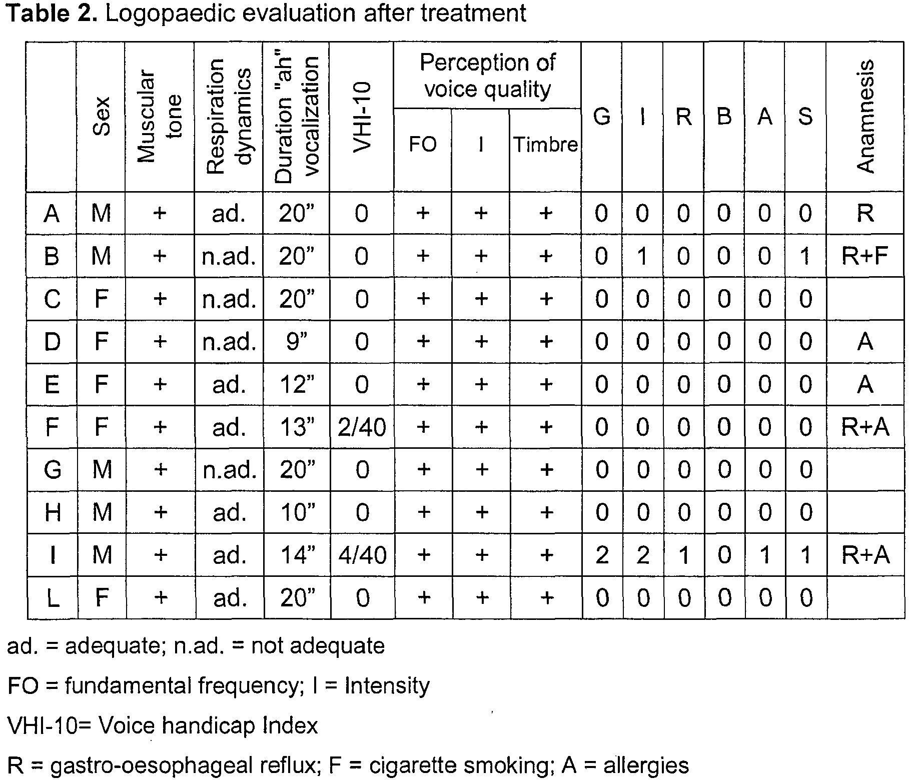

### 2) Evaluation of vocal quality using the Multi-Dimensional Voice Program (MDVP)

Referring to the appended drawings, analysis of results obtained showed that of the 5 individuals whose vocal parameters were inadequate, individual "I" showed an improvement of the Jitter and Shimmer values, but they remained altered, whereas individual "B" showed a more marked improvement, although still with altered values, while individuals "C", "F" and "G" all presented a clear improvement both of Jitter and of Shimmer.

Therefore, after administration of the composition of the invention, 8 individuals/10 showed normal values of Jitter and Shimmer, whereas in 2 individuals/10 these parameters, although improved, remained altered.

In the 5 individuals with adequate vocal parameters, the values of Jitter and Shimmer were improved in 5 individuals/5.

Conversely, the values of SPI decreased in all 10 individuals.

Thus, based on the tests that were carried out, it was observed that after taking the composition of the invention, all the parameters taken into consideration with FMDVP were improved. Moreover, these results were in agreement with the perceptive evaluation of the voice.

Based on spectrographic analysis of the voice, a clear improvement of vocal stability was observed both with respect to the fundamental frequency (FO) and the regularity of the formants. The harmonic spectrum appeared improved in 10 individuals out of 10. This result can probably be attributed to better cordal tonicity with better vocal performance. However, vocal attack appeared unchanged as none of the individuals had undergone logopaedic sessions for improving vocal emission. Moreover, some improvement was also observed in individuals with gastro-oesophageal reflux. This pathology definitely alters vocal quality, obliging the individual to exert intense vocal effort with overloading of the musculature to the detriment of the tension of the vocal ligament. In this connection, it is important to note that the use of the composition of the invention also provided a partial improvement in these individuals. So, it can be asserted that the composition of the invention potentiates vocal performance, allowing partial recovery of the capacity of vocal tension compromised by the underlying pathology. The composition of the invention therefore proved to be a surprisingly valid support both for subjects submitted to intense vocal loading, and for patients undergoing surgery, and for patients with acute and chronic inflammatory pathologies, advantageously speeding up the process of endemic healing to reintegrate the compartment of the fundamental nutrients for proper cordal function.

### Example 10

### Evaluation of efficacy- Comparative tests

Four groups of gelatin capsules were prepared, containing respectively:
i) the composition obtained according to Example 5,
ii) a composition of coenzyme Q10 (30 mg) and excipients,
iii) a composition of vitamin A (1 mg) and excipients, and
iv) a placebo composition of excipients only.

For the capsules of each type, a group of 9 individuals with cord hypotonia, suitably selected, was enrolled. Each individual was administered the capsules of the assigned type at a dose of two a day for a period of 10 consecutive days. Each individual underwent the following evaluations both pre-treatment and post- the treatment:
- logopaedic balance, completing the hetero-evaluation scale GIRBAS (Grade, Instability, Roughness, Breathiness, Asthenicity, Strain) and the Voice Handicap Index-10 self-assessment questionnaire
- Quantitative analysis of the vocal acoustic spectrum by Multi-Dimensional Voice Program with measurement of: Jitter (v.n. <1.04%), Shimmer (v.n. <3.81), Soft Phoniation Index (v.n. <14.12), harmonics/noise ratio, mean fundamental frequency and relative coefficients of variation. The study was conducted on vocal emissions between 55 and 65 dB and with sufficient spectrum stability. Background noise was monitored continuously and was kept below 30 dB.

### Results

The mean values post-treatment on the GIRBAS scale for each group are given in Table 3 below.

**Table 3. Logopaedic evaluation on the GIRBAS scale expressed as number and percentage of individuals improved post-treatment**

| Number of individuals | | | | | | |
|---|---|---|---|---|---|---|
| composition | G | I | R | B | A | S |
| comp. i) (Example 5) | 7 | 6 | 4 | 2 | 2 | 0 |
| comp. ii) (coenzyme Q10) | 3 | 2 | 1 | 1 | 1 | 0 |
| comp. iii) (vitamin A) | 2 | 1 | 0 | 0 | 0 | 0 |
| comp. iv) (placebo) | 1 | 0 | 0 | 0 | 0 | 0 |
| | | | | | | |

| % individuals | | | | | | |
|---|---|---|---|---|---|---|
| composition | G | I | R | B | A | S |
| comp. i) (Example 5) | **77.8%** | **66.7%** | **44.4%** | **22.2%** | **22.2%** | **0%** |
| comp. ii) (coenzyme Q10) | 33.3% | 22.2% | 11.1% | 11.1% | 11.1% | 0% |
| comp. iii) (vitamin A) | 22.2% | 11.1% | 0% | 0% | 0% | 0% |
| comp. iv) (placebo) | 11.1% | 0% | 0% | 0% | 0% | 0% |

From the results obtained using the GIRBAS scale and presented above, an extremely significant improvement was observed for all the parameters considered in the case of composition i) of the invention, i.e. comprising the combination coenzyme Q10+vitamin A. In contrast, composition ii) with only coenzyme Q10 achieved a modest improvement, limited to the parameters G and I, whereas for the other 4 parameters, the result was unsatisfactory. With respect to composition iii) comprising only vitamin A, the result was substantially comparable to that of the placebo, and therefore completely unsatisfactory.

Moreover, the following Table 4 presents the pre- and post-treatment values of VHI-10 (i.e. Voice Handicap Index-10), as well as the difference between the resultant values.

**Table 4. Logopaedic evaluation Voice Handicap Index-10 expressed as difference between pre- and post-treatment**

| VHI-10 | | | | |
|---|---|---|---|---|
| composition | | Pre- | Post- | Difference |
| comp. i) (Example 5) | Mean | 22.00 | 12.44 | 9.56 **(-43.45%)** |
| | S.D. | 8.87 | 9.72 | |
| comp. ii) (coenzyme Q10) | Mean | 19.44 | 16.00 | 3.44 (-17.69%) |
| | S.D. | 8.47 | 8.14 | |
| comp. iii) (vitamin A) | Mean | 19.56 | 18.22 | 1.33 (-6.79) |
| | S.D. | 7.78 | 7.05 | |
| comp. iv) (placebo) | Mean | 22.44 | 21.33 | 1.11 (-4.94) |
| | S.D. | 9.94 | 11.43 | |

From the results obtained by VHI-10 evaluation, presented above, an extremely significant reduction (i.e. -43.45%) was observed in vocal handicap in the case of composition i) of the invention, i.e. comprising the combination coenzyme Q10+vitamin A. In contrast, composition ii) with only coenzyme Q10 achieved a modest reduction, well below the preceding composition i). With respect to composition iii), comprising only vitamin A, the result was substantially comparable to that of the placebo, and therefore completely unsatisfactory.

The following Table 5 gives the post-treatment values obtained by Multi-Dimensional Voice Program (MDVP), expressed as the number and percentage of individuals improved.

**Table 5. Evaluation of vocal quality by Multi-Dimensional Voice Program (MDVP) expressed as number and percentage of individuals improved post-treatment**

| Number of individuals | | | |
|---|---|---|---|
| composition | JITTER | SHIMMER | SPI |
| comp. i) (Example 5) | 6 | 9 | 2 |
| comp. ii) (coenzyme Q10) | 2 | 4 | 2 |
| comp. iii) (vitamin A) | 1 | 1 | 1 |
| comp. iv) (placebo) | 0 | 1 | 1 |
| | | | |

| % individuals | | | |
|---|---|---|---|
| composition | JITTER | SHIMMER | SPI |
| comp. i) (Example 5) | **67%** | **100%** | **22%** |
| comp. ii) (coenzyme Q10) | 22% | 44% | 22% |
| comp. iii) (vitamin A) | 11% | 11% | 11% |
| comp. iv) (placebo) | 0% | 11% | 11% |

From the results obtained by MDVP, presented above, an extremely significant improvement was observed for all parameters considered in the case of composition i) of the invention, i.e. comprising the combination coenzyme Q10+vitamin A. In contrast, composition ii) with only coenzyme Q10 achieved a modest improvement, limited to the parameter Shimmer, whereas for the other 2 parameters, the result was unsatisfactory. With respect to composition iii) comprising only vitamin A, the result was substantially comparable to that of the placebo, and therefore completely unsatisfactory.

The results presented above clearly show the contribution demonstrated by the composition of the invention, for which unexpectedly significant and quantitative improvements were found. Moreover, it is shown that the combination of coenzyme Q10 and vitamin of the invention proved to be decidedly more effective than the individual components, as well as also more effective than the sum of the contributions of the two individual components, and it is therefore clear that the combination of the invention has a completely surprising synergistic effect. Therefore the composition of the present invention is particularly advantageous in the phonatory area (processes of production of language), in all cases of deterioration of the voice (dysphonia), including the professional and artistic voice and, in general, in states of fatigue of the laryngeal musculature due to a great variety of causes (functional and organic). Moreover, it proves particularly suitable as adjuvant in olfactory apparatus disorders characterized by anosmia and dysosmia.

## Claims

1. Composition consisting of coenzyme Q10, Vitamin A and pharmaceutically acceptable excipients.

2. The composition of Claim 1, wherein coenzyme Q10 is in an amount of 1-25 wt%, Vitamin A 0.3-10 wt%, on the composition weight.

3. The composition of Claim 2, wherein coenzyme Q10 is in an amount of 2-20 wt%, Vitamin A 0.5-8 wt%, on the composition weight.

4. The composition of Claim 3, wherein coenzyme Q10 is in an amount of 3-15 wt%, Vitamin A 1-6 wt%, on the composition weight.

5. The composition of Claim 4, wherein coenzyme Q10 is in an amount of 5-10 wt%, Vitamin A 1.5-5 wt%, on the composition weight.

6. The composition of any one of Claims 1-5, wherein said pharmaceutically acceptable excipients are colloidal silica, dibasic calcium phosphate, maltodextrins, sugar esters, microcrystalline cellulose, lactose, talc, magnesium stearate, calcium stearate, aluminum silicate or magnesium silicate, o mixtures thereof.

7. The composition of any one of Claims 1-6 for use as a medicament.

8. The composition of any one of Claims 1-6 for use in the treatment of phonatory apparatus disorders and olfactory apparatus disorders, such as anosmia and dysosmia.

9. The composition of any one of Claims 1-8, in the form of a unitary dose comprising 3.3-83 mg of Coenzyme Q10 and 1-26.6 mg of Vitamin A.

10. The composition of Claim 9, in the form of a unitary dose comprising 15-35 mg of Coenzyme Q10 and 5-15 mg of Vitamin A.

## Patentansprüche

1. Zusammensetzung bestehend aus Coenzym Q10, Vitamin A und pharmazeutisch annehmbaren Hilfsstoffen.

2. Zusammensetzung nach Anspruch 1, worin Coenzym Q10 in einer Menge von 1 bis 25 Gew.-%, Vitamin A in einer Menge von 0,3 bis 10 Gew.-% zugegen ist, bezogen auf das Gewicht der Zusammensetzung.

3. Zusammensetzung nach Anspruch 2, worin Coenzym Q10 in einer Menge von 2 bis 20 Gew.-%, Vitamin A in einer Menge von 0,5 bis 8 Gew.-% zugegen ist, bezogen auf das Gewicht der Zusammensetzung.

4. Zusammensetzung nach Anspruch 3, worin Coenzym Q10 in einer Menge von 3 bis 15 Gew.-%, Vitamin A in einer Menge von 1 bis 6 Gew.-% zugegen ist, bezogen auf das Gewicht der Zusammensetzung.

5. Zusammensetzung nach Anspruch 4, worin Coenzym Q10 in einer Menge von 5 bis 10 Gew.-%, Vitamin A in einer Menge von 1,5 bis 5 Gew.-% zugegen ist, bezogen auf das Gewicht der Zusammensetzung.

6. Zusammensetzung nach irgendeinem der Ansprüche 1 bis 5, worin die pharmazeutisch annehmbaren Hilfsstoffe kolloidale Kieselsäure, dibasisches Calciumphosphat, Maltodextrine, Zuckerester, mikrokristalline Cellulose, Lactose, Talkum, Magnesiumstearat, Calciumstearat, Aluminiumsilicat oder Magnesiumsilicat oder Mischungen davon sind.

7. Zusammensetzung nach irgendeinem der Ansprüche 1 bis 6 zur Verwendung als Medikament.

8. Zusammensetzung nach irgendeinem der Ansprüche 1 bis 6 zur Verwendung bei der Behandlung Störungen des Hör-Apparats und Störungen des Geruchs-Apparats, wie beispielsweise Anosmie und Dysosmie.

9. Zusammensetzung nach einem der Ansprüche 1 bis 8 in Form einer Einheitsdosis, die 3,3 bis 83 mg Coenzym Q 10 und 1 bis 26,6 mg Vitamin A umfasst.

10. Zusammensetzung nach Anspruch 9 in Form einer Einheitsdosis, die 15 bis 35 mg Coenzym Q 10 und 5 bis 15 mg Vitamin A umfasst.

## Revendications

1. Composition constituée de coenzyme Q10, de vitamine A et d'excipients pharmaceutiquement acceptables.

2. Composition selon la revendication 1, dans laquelle la coenzyme Q10 est présente en une quantité de 1 à 25 % en poids, la vitamine A de 0,3 à 10 % en poids, par rapport au poids de la composition.

3. Composition selon la revendication 2, dans laquelle la coenzyme Q10 est présente en une quantité de 2 à 20 % en poids, la vitamine A de 0,5 à 8 % en poids, par rapport au poids de la composition.

4. Composition selon la revendication 3, dans laquelle la coenzyme Q10 est présente en une quantité de 3 à 15 % en poids, la vitamine A de 1 à 6 % en poids, par rapport au poids de la composition.

5. Composition selon la revendication 4, dans laquelle la coenzyme Q10 est présente en une quantité de 5 à 10 % en poids, la vitamine A de 1,5 à 5 % en poids, par rapport au poids de la composition.

6. Composition selon l'une quelconque des revendications 1 à 5, dans laquelle lesdits excipients pharmaceutiquement acceptables sont la silice colloïdale, le phosphate de calcium dibasique, les maltodextrines, les esters de sucre, la cellulose microcristalline, le lactose, le talc, le stéarate de magnésium, le stéarate de calcium, le silicate d'aluminium ou le silicate de magnésium, ou des mélanges de ceux-ci.

7. Composition selon l'une quelconque des revendications 1 à 6, pour son utilisation en tant que médicament.

8. Composition selon l'une quelconque des revendications 1 à 6, pour son utilisation dans le traitement de troubles de l'appareil phonatoire et de troubles de l'appareil olfactif, tels que l'anosmie et la dysosmie.

9. Composition selon l'une quelconque des revendications 1 à 8, sous la forme d'une dose unitaire comprenant de 3,3 à 83 mg de coenzyme Q10 et de 1 à 26,6 mg de vitamine A.

10. Composition selon la revendication 9, sous la forme d'une dose unitaire comprenant de 15 à 35 mg de coenzyme Q10 et de 5 à 15 mg de vitamine A.
